(19) Europäisches Patentamt
European Patent Office
Office européen des brevets

(11) **EP 4 289 366 A1**

(12) **EUROPEAN PATENT APPLICATION**

(43) Date of publication:
**13.12.2023 Bulletin 2023/50**

(21) Application number: **22305850.4**

(22) Date of filing: **10.06.2022**

(51) International Patent Classification (IPC):
**A61B 8/08** *(2006.01)*     **A61B 8/12** *(2006.01)*
**A61B 8/00** *(2006.01)*

(52) Cooperative Patent Classification (CPC):
**A61B 8/12; A61B 8/0841; A61B 8/4245;**
**A61B 8/4477;** A61B 8/0808

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Robeaute**
**75002 Paris (FR)**

(72) Inventors:
• **DUPLAT, Bertrand**
 **75002 Paris (FR)**

• **FRANCOIS, Quentin**
 **75002 Paris (FR)**
• **ZARADER, Pierre**
 **95590 Presles (FR)**
• **COUTURE, Olivier**
 **75016 Paris (FR)**
• **HALIYO, Sinan**
 **75006 Paris (FR)**
• **RÉGNIER, Stéphane**
 **75006 Paris (FR)**

(74) Representative: **Icosa**
**83 avenue Denfert-Rochereau**
**75014 Paris (FR)**

(54) **TRACKING SYSTEM FOR 3D TRANSCRANIAL TRACKING OF A MEDICAL DEVICE**

(57)     The present invention relates to a tracking system (1) and a method for 3D tracking of a medical device, equipped of at least one ultrasound sensor, inside an anatomical region of a subject comprising at least one layer of a first tissue type at least partially surrounding a volume comprising at least one second tissue type, wherein the properties of ultrasounds propagation in the first and second tissue type are different.

FIG. 1

EP 4 289 366 A1

## Description

## FIELD OF INVENTION

The present invention relates to the field of tracking position of medical devices for assisting during intervention on patient. The present invention relates notably to the use of ultrasounds for the localization and tracking of a medical device inside the body of a patient.

## BACKGROUND OF INVENTION

Recent breakthroughs in microtechnologies open the possibility to navigate medical microdevices inside the human body to reach deep and hard-to-access structures. For safety purposes, this microrobot should be as autonomous as possible and most preferably be controlled from outside the patient's body in a contactless manner. This micro-device thus needs a contactless localization system defining an internal referential, in order to be tracked and precisely located while moving inside the target body part. This system should also be able, in order to enable a surgeon to be in full control of the situation, to offer a precise 3D localization of said microrobot inside the target body part.

There is a need for improving the tracking system and especially the localization for this kind of microrobots in correspondence to the anatomy of the target body part. The position of the microrobot with respect to anatomic features, such as functional areas, vessels or nerve, is of primary importance since it will define the path and target point of the robot, inside the target body part. A 3D imaging modality is thus necessary to envision these features and permit path planning. Moreover, the imaging modality and the microrobot positioning has to be co-registered with a precision better than 1 mm. Particularly, in neurosurgery, medical microdevices need a non-invasive tracking system at least as accurate as their size.

In this context, there is the need of developing a system for tracking a medical device with submillimetric accuracy.

## SUMMARY

This invention thus relates a tracking system for estimating a 3D position of a medical device, equipped of at least one ultrasound sensor, inside an anatomical region of a subject comprising at least one layer of a first tissue type at least partially surrounding a volume comprising at least one second tissue type, wherein the properties of ultrasounds propagation in the first and second tissue type are different, said system comprising:

- at least one input configured to receive a position for each of at least three external ultrasound sensors with respect to the at least one first tissue layer, information on a geometry of the at least one first tissue layer and a 3D map of speed of ultrasounds in the at least one first tissue layer and the volume of at least one second tissue;

- at least one processor configured to:

  o obtain a current position $(P_{3D}^j)$ of the medical device inside the volume of at least one second tissue;
  o perform location computation comprising:

  - using the obtained current position $(P_{3D}^j)$ to calculate a current elevation angle $(\theta_i^j)$ and a current lateral angle $(\varphi_i^j)$ of the medical device with respect to each external ultrasound sensor, and using said current elevation angle $(\theta_i^j)$ and current lateral angle $(\varphi_i^j)$ to estimate a current path $(l_i^j)$ traveled by the ultrasounds through the at least one first tissue layer between each external ultrasound sensor and the medical device,
  - for each external ultrasound sensor, obtaining an associated current local speed in the first tissue $(c_i^j)$ using the estimated current path $(l_i^j)$ and said 3D map of speed in the first tissue;

  - calculating a current distance $(d_i^j)$ between each external ultrasound sensor and the medical device by considering the current local speed in the at least one first tissue layer $(c_i^j)$ associated to the corresponding external ultrasound sensor and the estimated current path $(l_i^j)$ associated to the corresponding external ultrasound sensor, a speed of ultrasounds in the at least one second tissue and a measured time of propagation of the ultrasounds between each external ultrasound sensor and the medical device;

  - estimating a new position of the medical device inside the volume of at least one second tissue using the current distance $(d_i^j)$ between each external ultrasound sensor (Si) and the medical device;

   o repeat performing location computation using the new position as current position $(P_{3D}^{j+1})$, until an exit criterion is satisfied;

   o at least one output configured to provide the estimated position of the medical device satisfying the exit criterion.

According to other advantageous aspects of the invention, the device comprises one or more of the features described in the following embodiment, taken alone or in any possible combination:

- calculating the current distance $(d_i^j)$ comprises using numerical simulations of the propagation of the ultrasounds in the at least one first tissue layer and the volume of at least one second tissue of the subject, said numerical simulations being based on medical imaging data of at least one portion of the anatomical region of the subject,

- obtaining a current position $(P_{3D}^j)$ of the medical device inside the volume of second tissue for the first iteration comprises:

   i. obtaining a thickness (tf) of the at least one first tissue layer at the position of each external ultrasound sensor using the 3D geometry of the at least one first tissue layer of the subject;

   ii. for each external ultrasound sensors, using the 3D map of speed in the first tissue to obtain a local speed in the first tissue $(c_i^1)$, corresponding to the speed of propagation of ultrasounds through the at least one first tissue layer in correspondence to the external ultrasound sensor position;

   iii. for each external ultrasound sensor, estimating a first distance $(d_i^1)$ between the external ultrasound sensor and the medical device based on the corresponding obtained local speed in the at least one first tissue layer $(c_i^1)$ propagating through the at least one first tissue layer for a path equal to the corresponding obtained thickness $(t_i^1)$, the speed of ultrasound in the second tissue and a measured time of propagation of the ultrasounds between each external ultrasound sensor (Si) and the medical device;

wherein the current position $(P_{3D}^j)$ of the medical device inside the volume of at least one second tissue, for the first iteration, is obtained using the estimated first distances $(d_i^1)$ between the medical device and each external ultrasound sensor,

- obtaining a current position $(P_{3D}^j)$ of the medical device inside the volume of at least one second, for the first iteration, comprises using medical imaging data,

- the measured time of propagation of the ultrasounds between each external ultrasound sensor and the medical device is based on a direct time of flight measure measured using each external ultrasound sensor and the at least one ultrasound sensor of the medical device,

- the exit criterion is configured to stop the iterations when for a given number of iterations the difference between the current position $(P_{3D}^j)$ and the estimated new position is inferior to a predefined threshold,

- the external ultrasound sensors are ultrasound emitters configured to modify the direction of emission of the ultrasounds and the medical device is equipped with an ultrasound receiver, and wherein the at least one processor is further configured to use the estimated position of the medical device so as to modify the direction of emission of the ultrasounds in order to focus the ultrasounds of the at least three external ultrasound sensors in the position of the medical device,

- the at least one layer of a first tissue type is a skull of the subject and the volume of at least on second tissue is a brain of the subject, or the at least one layer of a first tissue type is layer of fat and the at least one volume is a liver.

In addition, the disclosure relates to a computer program comprising software code adapted to perform a method for estimating a position and 3D tracking of a medical device compliant with any of the above execution modes when the program is executed by a processor.

The present invention has for further object a computer-implemented method for estimation of a 3D position of a medical device, equipped of at least one ultrasound sensor, inside an anatomical region of a subject comprising at least one layer of a first tissue type at least partially surrounding a volume comprising at least one second tissue type, wherein the properties of ultrasounds propagation in the first and second tissue type are different, said system comprising:

- receiving a position for each of at least three external ultrasound sensors with respect to the at least one first tissue layer, information on a geometry of the at least one first tissue layer and a 3D map of speed of ultrasounds in the at least one first tissue layer and the volume of at least one second tissue;

- obtaining a current position $(P_{3D}^{j})$ of the medical device inside the volume of second tissue;

- performing location computation comprising:

  ▪ using the obtained current position $(P_{3D}^{j})$ to calculate a current elevation angle $(\theta_i^j)$ and a current lateral angle $(\varphi_i^j)$ of the medical device with respect to each external ultrasound sensor, and use said current elevation angle $(\theta_i^j)$ and current lateral angle $(\varphi_i^j)$ to estimate a current path $(l_i^j)$ traveled by the ultrasounds through the at least one first tissue layer between each external ultrasound sensor and the medical device

  ▪ for each external ultrasound sensor (Si), estimating an associated current local speed in the first tissue $(c_i^j)$ using the current path $(l_i^j)$ and said 3D map of speed in the first tissue;

  ▪ calculating a current distance $(d_i^j)$ between each external ultrasound sensor (Si) and the medical device by considering the current local speed in the at least one first tissue layer $(c_i^j)$ associated to the corresponding external ultrasound sensor and the estimated current path $(l_i^j)$ associated to the corresponding external ultrasound sensor, a speed of ultrasounds in the at least one second tissue and a measured time of propagation of the ultrasounds between each external ultrasound sensor and the medical device;

  ▪ estimating a new position of the medical device inside the volume of at least one second tissue using the current distance $(d_i^j)$ between each external ultrasound sensor and the medical device;

- repeat performing location computation using the new position as current position $(P_{3D}^{j+1})$, until an exit criterion has been satisfied.

Said method may comprise one or more of the following steps:

- calculating the current distance $(d_i^j)$ comprises using numerical simulations of the propagation of the ultrasounds in the first type tissue and second type tissues of the subject, said numerical simulations being based on medical imaging data of at least one portion of the anatomical region of the subject,

- obtaining a current position (PiD) of the medical device (M) inside the volume of second tissue comprises:

  iv. obtaining a thickness (tf) of the at least one first tissue layer at the position of each external ultrasound sensor (Si) using the 3D geometry of the at least one first tissue layer of the subject;
  v. for each external ultrasound sensors (Si), using the 3D map of speed first tissue to obtain a local speed in the first tissue $(c_i^1)$, corresponding to the speed of propagation of ultrasounds through the at least one first tissue layer in correspondence to the external ultrasound sensor position;
  vi. for each external ultrasound sensor, estimating a first distance $(d_i^1)$ between the external ultrasound sensor and the medical device based on the corresponding obtained local speed in the first tissue $(c_i^1)$ propagating through the at least one first tissue layer for a path equal to the corresponding obtained thickness $(t_i^1)$, the speed of ultrasound in the second tissue and a measured time of propagation of the ultrasounds between each external ultrasound sensor and the medical device;

  wherein the current position $(P_{3D}^{j})$ of the medical device inside the volume of at least one second tissue is obtained using the estimated first distances $(d_i^1)$ between the medical device and each external ultrasound sensor.

- the exit criterion is configured to stop the iterations when for a given number of iterations the difference between the current position $(P_{3D}^{j})$ and the new position $(P_{3D}^{j+1})$ is inferior to a predefined threshold,

- the external ultrasound sensors are ultrasound emitters configured to modify the direction of emission of the ultrasounds and the medical device is equipped with an ultrasound receiver, and wherein the at least one processor is further configured to use the new position $(P_{3D}^{j})$ so as to modify the direction of emission of the ultrasounds in order to focus the ultrasounds of the at least three skull ultrasound sensors (Si) in the position of the medical device.

The present disclosure further pertains to a non-transitory program storage device, readable by a computer, tangibly embodying a program of instructions executable by the computer to perform a method for estimating a position and 3D tracking of a medical device, compliant with the present disclosure.

Such a non-transitory program storage device can be, without limitation, an electronic, magnetic, optical, electromagnetic, infrared, or semiconductor device, or any suitable combination of the foregoing. It is to be appreciated that the following, while providing more specific examples, is merely an illustrative and not exhaustive listing as readily appreciated by one of ordinary skill in the art: a portable computer diskette, a hard disk, a ROM, an EPROM (Erasable Programmable ROM) or a Flash memory, a portable CD-ROM (Compact-Disc ROM).

## DEFINITIONS

In the present invention, the following terms have the following meanings:

In the present invention, the following terms have the following meanings:

The terms **"adapted"** and **"configured"** are used in the present disclosure as broadly encompassing initial configuration, later adaptation or complementation of the present device, or any combination thereof alike, whether effected through material or software means (including firmware).

The term **"processor"** should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

## BRIEF DESCRIPTION OF THE DRAWINGS

**Figure 1** is a block diagram representing schematically a particular mode of a system for estimating a 3D position of a medical device.

**Figure 2** is schematic representation of the tracking system and the medical device and the external ultrasound sensors disposed with respect to an anatomical region of the subject.

**Figure 3** is a block diagram representing schematically a particular mode of the module for obtaining a first current position.

**Figure 4** is a block diagram representing schematically a particular mode of the module for performing the localization computation loop.

**Figure 5** is a schematic representation of the current elevation and lateral angle used to estimate the current path traveled by the ultrasounds through the at least one first tissue layer.

**Figure 6** is a schematic representation of the use of the tracking system for estimating a position and 3D tracking of a medical device positioned inside the brain of a subject.

**Figure 7** is a flow chart showing successive steps executed with the device for 3D tracking of figure 1.

**Figure 8** diagrammatically shows an apparatus integrating the functions of the device for 3D tracking of figure 1.

## ILLUSTRATIVE EMBODIMENTS

The present description illustrates the principles of the present disclosure. It will thus be appreciated that those skilled in the art will be able to devise various arrangements that, although not explicitly described or shown herein, embody the principles of the disclosure and are included within its scope.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Thus, for example, it will be appreciated by those skilled in the art that the block diagrams presented herein may represent conceptual views of illustrative circuitry embodying the principles of the disclosure. Similarly, it will be appreciated that any flow charts, flow diagrams, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

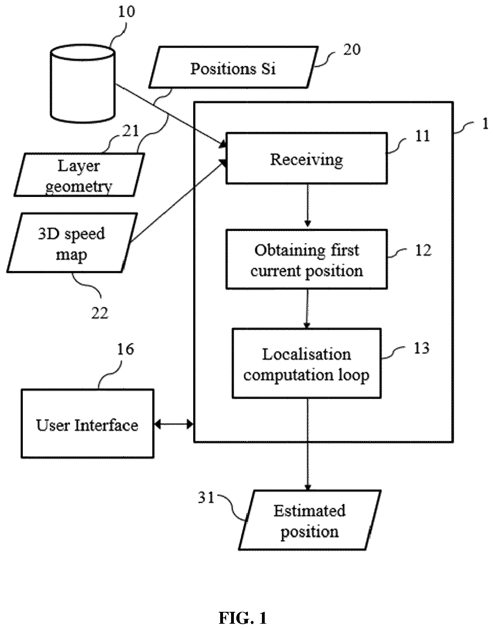

The present disclosure will be described in reference to a particular functional embodiment of a tracking system 1 for estimating a position and 3D tracking of a medical device, as illustrated on **Figure 1.**

The medical device of the present invention may be a surgical microrobot having dimensions of the order of the millimeter. Other medicals devices that may be positioned inside the patient thanks to the system and the method of the present invention are catheters, surgical probes, biopsy needles, laparoscopes or any other surgical instruments to be inserted in the body of the patient and that necessitate a tracking during use.

The medical device M is generally introduced in the anatomical structure of the subject to inspect or to treat during a surgery. In one example, a hole may be provided in the first tissue layer, notably when the first tissue type is bone tissue, to introduce the medical device M directly in the second tissue volume.

The tracking system 1 is adapted to provide an estimation of the position of the medical device m in the anatomical region at a given time. When the medical device M moves inside the anatomical region, the tracking system 1 is configured to provide the position of the medical device M as a function of time. As will be explained more in details in the following paragraphs, the particular choices of steps implemented by the processor allow to obtain the information on the actual position of the medical device M in real time (i.e.; 20 positions estimation per second).

Though the presently described tracking system 1 is versatile and provided with several functions that can be carried out alternatively or in any cumulative way, other implementations within the scope of the present disclosure include devices having only parts of the present functionalities.

The tracking system 1 is advantageously an apparatus, or a physical part of an apparatus, designed, configured and/or adapted for performing the mentioned functions and produce the mentioned effects or results. In alternative implementations, any of the tracking system 1 is embodied as a set of apparatus or physical parts of apparatus, whether grouped in a same machine or in different, possibly remote, machines. The tracking system 1 may e.g. have functions distributed over a cloud infrastructure and be available to users as a cloud-based service, or have remote functions accessible through an API.

In what follows, the modules are to be understood as functional entities rather than material, physically distinct, components. They can consequently be embodied either as grouped together in a same tangible and concrete component, or distributed into several such components. Also, each of those modules is possibly itself shared between at least two physical components. In addition, the modules are implemented in hardware, software, firmware, or any mixed form thereof as well. They are preferably embodied within at least one processor of the tracking system 1.

The tracking system 1 comprises a module 11 for receiving the multiple inputs necessary for the steps to be implemented. Notably, the module 11 is configured to receive a position 20 for each of at least three external ultrasound sensors Si with respect to the at least one first tissue layer, information on a geometry 21 of the at least one first tissue layer and a 3D map of speed of ultrasounds in the at least one first tissue layer and the volume of at least one second tissue 22. These inputs 20, 21 and 22 may have been stored in one or more local or remote database(s) 10. The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk).

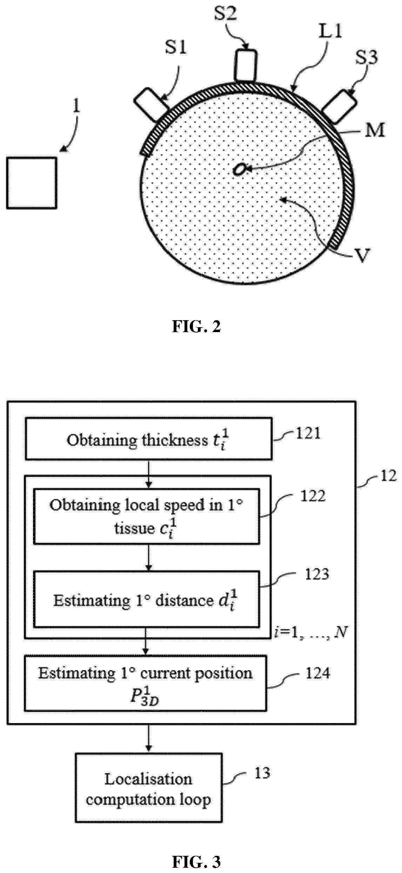

As illustrated in Figure 2, the at least three external ultrasound sensors Si (for $i$ = 1, ..., N, herein N is equal or superior to three) are positioned at three different locations with respect to the anatomical region and may have been put in contact with the at least one layer of first tissue type L1. Depending from the anatomical region, said external ultrasound sensors Si may be rigidly fixed to a portion of the anatomical region or not. For example, when the first tissue type is bone tissue, the external ultrasound sensors Si may be rigidly fixed onto the bone or to a surgical tool that does not change of relative position with respect to the anatomical structure. It might as well be possible to position the at least three external ultrasound sensors Si on a portion of anatomical structure that may drift, swell or be modified during the surgery so that the positions of the at least three external ultrasound sensors Si with respect to the first tissue layer L1 or the second tissue volume V change during the surgery. In that case, the at least three external ultrasound sensors Si may be each equipped of fiducials coupled to a navigation system capable of tracking the position of each external ultrasound sensors Si with respect to the first tissue layer L1. The tracking information may be used to correct in real time the position of each external ultrasound sensors Si so as not to lose precision in the 3D tracking of the medical device M.

When the external ultrasound sensors Si are rigidly fixed with respect to the anatomical structure (i.e., their position will not change during the surgery), their position 20 may have been obtained previous to the beginning of the surgery (but after fixing them on the subject) using medical imaging technics such as a CT scan or MRI imaging. In the alternative case when no rigid fixation is possible and a navigation system is used, the position 20 of the external ultrasound sensors Si may be directly measured in the surgical theater right at the beginning of the surgery and may be tracked all the long of the surgery.

The information on a geometry 21 of the at least one first tissue layer and, eventually, the second tissue volume of the anatomical structure may be derived from medical imaging data such as the CT-scan and/or MRI depending on the nature of the first and at least one second tissue type. The information on the geometry 21 may for example comprise the thickness of the first tissue layer at each point of its surface. Plus second layer

The 3D map of speed of ultrasounds in the at least one first tissue layer and the volume of at least one second tissue 22 may be derived from CT scan imaging data or any other medical imaging technics known by the skilled artisan. Indeed, the actual speed of sound (i.e., ultrasound) varies depending on tissue structural characteristics. Using the 3D map of speed of ultrasounds, instead of just an approximation of sound speed for the first tissue type and the second tissue type allows to obtain a better tracking precision.

The reception module 11 may be further configured to be in communication, notably via a wireless communication network, with the medical device M and the external ultrasound sensors Si. The reception module 11 may be therefore configured to receive in real time the measurements performed by the ultrasound sensor of the medical device M and the external ultrasound sensors Si. In one example, the ultrasound sensor of the medical device M is an ultrasounds emitter and the external ultrasound sensors Si are ultrasounds receivers, or inversely the ultrasound sensor of the medical device M is a receiver and the external ultrasound sensors Si are ultrasound emitters. In both cases, the ultrasound sensor of the medical device M and the external ultrasound sensors Si allow to measure the time took by sound to propagate between the medical device and the external ultrasounds sensors. This configuration of the external ultrasound sensors Si and of the medical device sensor is particularly advantageous with respect to the use of a standard ultrasounds probe configured to both emit and receive ultrasounds, in the case when the first type tissue strongly attenuates the intensity of the ultrasounds such as for bone tissue. Indeed, by separating the emitter and the receiver, the emitted ultrasounds only passe thought the first tissue layer once before being received by the receiver. Inversely, when a standard probe is used the ultrasounds passe the first layer a first time, then are reflected by the tissues in the volume and by the medical device and said reflected ultrasounds, that traverse a second time the first tissue layer, are detected by the receiver. However, the signal-to-noise ratio of these ultrasounds is degraded due to the double passage in the first tissue layer.

The tracking system 1 further comprises an initialization module 12 for obtaining a first position of the medical device M inside the volume of at least one second tissue which is then used in localization module 13 as starting position for the location computation loop.

According to one embodiment, the initialization module 12 is configured to receive imaging data (i.e., obtained from medical imaging technics others than the ultrasounds sensors) and analyze the imaging data in order to obtain a first approximative calculation of the position $P_{3D}^1$ of the medical device M.

According to one alternative embodiment illustrated in Figure 3, the initialization module 12 is configured to use the measure obtained from the external ultrasounds' sensors Si and the ultrasound sensor of the medical device M to obtain an approximative calculation of the position $P_{3D}^1$ (i.e., first current position) of the medical device M.

The initialization module 12 may be configured to perform the steps 121, 122 and 123 for each of the external ultrasound sensor Si. The result of the three steps obtained for each external ultrasound sensor Si is then combined in step 124. The step 121 is configured to obtain thicknesses $t_i^1$ of the at least one first tissue layer at the position of each external ultrasound sensor Si. Said thicknesses $t_i^1$ is obtained using the received 3D geometry of the at least one first tissue layer of the subject. The average thickness is defined as the length of the first tissue layer in the normal direction to the area in front of the which the external ultrasounds sensors Si are placed.

The step 122 is configured to obtain a local speed in the first tissue type $c_i^1$ which corresponds to the speed of propagation of ultrasounds through the at least one first tissue layer in correspondence to the external ultrasound sensor position. The local speed in the first tissue type $c_i^1$ is obtained using the 3D map of speed in the first tissue. Indeed, as explained above, the speed of sound in one tissue type may variate depending on several physical parameters. Therefore, the received position of the external ultrasound sensors Si is used to evaluate the speed of sound propagating in the first tissue layer in front of each external ultrasound sensors Si.

The step 123 consists in estimating a first distance $d_i^1$ between each of the external ultrasound sensor Si and the medical device M. In order to obtain these first distances estimations $d_i^1$, it is necessary to use the information on the speed of the ultrasounds through the tissues present between the real position of the medical device M and the external ultrasound sensor Si considered and the time taken may the ultrasound to propagate between the medical device M and the external ultrasound sensor Si considered. Two speed values have to be considered: the obtained local speed in the first tissue type $c_i^1$ and the speed of ultrasound in the second tissue type, which may be obtained as well from the 3D map of speed. For the speed of ultrasound in the second tissue type, an average value may be considered. Concerning the time measure, the module 12 is configured to receive a measured time of propagation of the ultrasounds between each external ultrasound sensor Si and the medical device M. Said measured time may be obtained from a Time-of-Flight measure between the medical device M and the external ultrasound sensor Si considered.

The first distance for each external ultrasound sensor Si may be calculated using the following formula:

$$d_i^1 = t_i^1 + v_2\left(T_i - \frac{t_i^1}{c_i^1}\right)$$

, wherein the $v_2$ is the average speed of sound in the second type tissue and $T_i$ is the measured time of propagation of the ultrasounds between each external ultrasound sensor Si and the medical device M.

After the determination of the first distances $d_i^1$ between each external ultrasound sensor Si and the medical device M, the step 124 is configured to calculate the approximation of the first current position $P_{3D}^1$. This first current position $P_{3D}^1$ is only an approximation of the 3D position on the medical device M as it is calculated using the assumption that the propagation path traversed by the ultrasound in the first tissue layer is equal to the average thickness of the first tissue layer, however depending on the real position of the medical device said propagation path may be longer.

This first current position $P_{3D}^1$ (i.e., current position $P_{3D}^j$ to be used for the first iteration, j = 1, for initialization of localization loop) is obtained by triangulation or trilateration using the estimated first distances $d_i^1$ between the medical device M and each external ultrasound sensor Si.

In one example, the measured time $T_i$ of propagation of the ultrasounds between each external ultrasound sensor Si and the medical device M is based on a direct time of flight measure measured using each external ultrasound sensor Si and the at least one ultrasound sensor of the medical device M.

According to one alternative embodiment, notably when the tracking system have been used to estimate at least one 3D position of the medical device M, the module 12 is configured to that the first current position $P_{3D}^1$ is obtained as the last estimated position of the medical device M satisfying the exit criterion.

The tracking system 1 further comprises a location computation loop module 13 for performing location computation. Said location computation loop module 13 is configured to receive as input the first current position $P_{3D}^1$ obtained with the initialization module 12. The module 13 set this first current position $P_{3D}^1$ as the initial value of the current position for the first iteration of the location computation loop (j =1). Indeed, the present invention uses a first approximative estimation of the current position as information to improve the precision in the next calculation of the current position for one real given position of the medical device till the calculation converges to an optimal estimation of the current position of the medical device which has only a submillimetric error in the estimation. More precisely, the use of the steps of module 13 allows to reduce the error from approximately 1 - 2 mm (i.e., 1 mm if only the thickness of the first tissue layer in front of each external ultrasounds sensor is considered and 2mm if only one average thickness of the first tissue layer is considered) to an error between 100 and 900 micrometers.

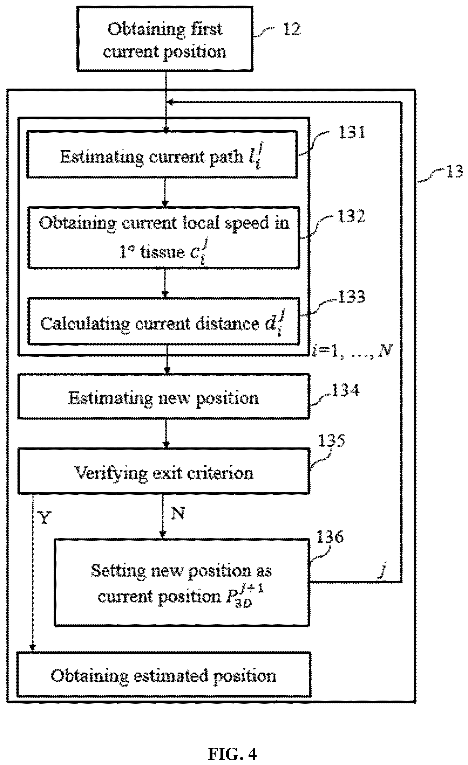

As shown in Figure 4, the module 13 may be configured to implement successive steps 131 to 136, wherein steps 131 to 133 are performed with respect to each external ultrasound sensor Si, until a predefined exit criterion is verified. The current position used at step 131 for the first iteration ($j$ = 1) is the first current position obtained by the module 12 while *for j >2* the current position used at step 131 is the new (current) position obtained as result of the iteration *j-1*.

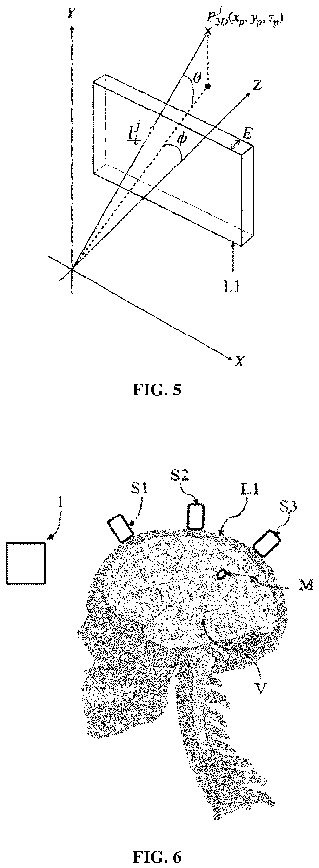

The step 131 is configured to calculate the current elevation angle $\theta_i^j$ and the current lateral angle $\varphi_i^j$ of the medical device M with respect to each external ultrasound sensor Si using the obtained current position $P_{3D}^j$ (see figure 5). Then in step 131, the current elevation angle $\theta_i^j$ and a current lateral angle $\varphi_i^j$ are used to estimate a current path $l_i^j$ traveled by the ultrasound through the at least one first tissue layer between each external ultrasound sensor Si and the medical device M.

The following step 132 uses the current path $l_i^j$, estimated in step 131, to know with higher precision in which part of the first tissue layer the ultrasounds passes to reach each of the external ultrasound sensor Si. Then, this information is used in combination with the 3D map of speed in the first tissue to obtain the current local speed of ultrasounds in the first tissue $c_i^j$ when traversing the first tissue layer according to the current path $l_i^j$ obtained for each of the external ultrasound sensor Si. This step advantageously allows to take in consideration heterogeneities in the structure of the first tissue layer that could lead to a change in the speed of ultrasounds in the first tissue type. This is the case of bone tissue. Indeed, not only the density of the skull changes from patient to patient, as it depends on age and others factors (e.g., pathologies), but also the skull of one patient presents different density depending on the region of the skull.

The successive step 133 is configured to calculating the current distance $d_i^j$ (i.e., for the *j*th iteration) between each external ultrasound sensor Si and the medical device M which is inside the volume of the at last one second tissue. In order to calculate the current distances $d_i^j$, the step 133 uses the estimated current path $l_i^j$ associated to the corresponding external ultrasound sensor Si, the current local speed in the at least one first tissue layer $c_i^j$ associated to the corresponding external ultrasound sensor Si (i.e., obtained at step 132), a speed of ultrasounds in the at least one second tissue and the measured time of propagation of the ultrasounds between each external ultrasound sensor Si and the medical device M. Notably, the current path $l_i^j$ is used to estimate the length traversed by the ultrasounds inside the second tissue volume and the length traversed by the ultrasounds inside the first tissue layer. In one example, for the speed of ultrasounds in the at least one second tissue it is used an average speed value. The formula used to estimate the current distances $d_i^j$ is:

$$d_i^j = l_i^j + v_2 \left( T_i - \frac{l_i^j}{c_i^j} \right).$$

Alternatively, the step 133 is configured to use numerical simulations of the propagation of the ultrasound in the at least one first tissue layer and the volume of at least one second tissue of the subject in order to calculate the current distances $d_i^j$ for each external ultrasound sensor Si. These numerical simulations can take into account the phenomena of reflection, refraction, diffraction, diffusion of ultrasound in the at least one first tissue layer and the volume of at least one second tissue of the subject. Said numerical simulations are based on medical imaging data of at least one portion of the anatomical region of the subject.

The step 134 is then configured to estimate new position of the medical device M (i.e., new corrected position) inside the volume of at least one second tissue using the current distances $d_i^j$ between each external ultrasound sensor Si and the medical device M calculated at the step 133. The new position is calculated by triangulation or trilateration using the current distances $d_i^j$.

A step 135 is then implemented in order to verify whether the estimated new position satisfies the predefined exit criteria. When the exit criterion is not satisfied, the step 135 is configured to define as the current position for the next iteration *j+1* the new position, so that the

current position $P_{3D}^{j+1}$ received at input of the step 131 is the new position estimated at the iteration $j$. The step 135 is configured, in response to the satisfaction of the criterion, to stop the iterations of the module 13 and define a final estimated position 31 that satisfy the exit criterion.

The exit criterion may simply consist in verifying that one iteration of the step 131 to 134 has been executed. In this example, the final estimated position 31 is the first new position estimated at the end of the first iteration. In the same way, the exit criterion my be configured to be satisfied when a predefined number of iterations is completed.

Alternatively, the exit criterion may be configured to stop the iterations when for a given number of iterations, for example three iterations, the difference between the current position $P_{3D}^{j}$ and the new position is inferior to a predefined threshold, so that when the result converges to one value the iterations are stopped. In this case, the final estimated position 31 is defined as the new estimated position of the $j^{th}$ iteration for which the exit criterion is satisfied.

As described above, the external ultrasound sensors Si may ultrasound emitters and the medical device may be equipped with an ultrasound receiver. The external ultrasound sensors Si may be ultrasound emitters configured to modify the direction of emission of the ultrasounds. In this case, the system 1 is further configured to comprise a module 14, not represented, configured to wirelessly communicate to the external ultrasound sensors in order to provide instructions to modify the direction of emission of the ultrasounds of each external ultrasound sensors Si according to the final estimated position of the medical device M. Advantageously, the direction of emission of the external ultrasound sensors Si is modified so as to focus the ultrasounds on the medical device M which allows to improve the signal to noise ratio and therefore the precision of 3D position reconstruction. This embodiment of the invention is particularly advantageous when a low number (i.e., three) of external ultrasound sensors Si is used.

The tracking system 1 is interacting with a user interface 16, via which information can be entered and retrieved by a user. The user interface 16 includes any means appropriate for entering or retrieving data, information or instructions, notably visual, tactile and/or audio capacities that can encompass any or several of the following means as well known by a person skilled in the art: a screen, a keyboard, a trackball, a touchpad, a touchscreen, a loudspeaker, a voice recognition system.

Therefore, the tracking system 1 of the present invention allows to estimate the position of the medical device inside the body of a patient at a given position. The tracking system 1 may be as well used to track in real time the position of the medical device that is moving inside the second tissue volume. By repeating the steps described above for multiple real positions of the medical device it is possible to monitor the trajectory of the medical device M. Furthermore, the implementation of the steps is rapid enough to allow to obtain the estimated position 31 of the medical device M twenty times per seconds with correspond to a real time tracking of the medical device.

Figure 6 show an example of application of the system 1 wherein the external ultrasounds sensors S1, S2 and S3 are three and are fixed on the head of a patient. The first tissue layer L1 in this case is the skull while the second tissue volume V are the soft tissue of the brain. The medical device M represented in this figure is a microrobot configured to travel in the brain of the subject.

In this case scenario, as in other practical cases, the ultrasounds have to travel not only through the skull but also through the skin which can have different properties of ultrasounds propagation than the first and second tissue type. As the skin is a low-density tissue, the fact that the presence of the skin layer ignored and only the first layer of tissue is considered in the calculation does not jeopardize the results, which as disclosed above, have submillimetric precision.

However, the module 12 and module 13 may be as configured to take in consideration the presence of a second layer of a third tissue different (or not) from the first and second tissue. The skilled artisan would know how to take into consideration the presence of a second layer covering the first layer or positioned between the first layer and the volume V in the steps implemented in module 12 and 13. This embodiment would allow to reduce even more the precision of the 3D localization.

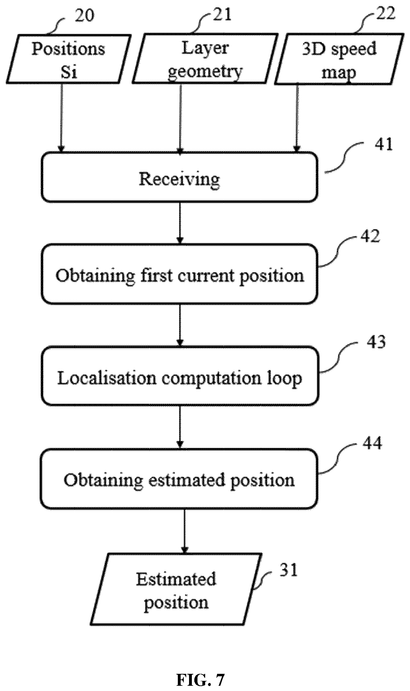

In its automatic actions, the tracking system 1 may for example execute the following process (Figure 7):

- receiving the position 20 for each of at least three external ultrasound sensors Si with respect to the at least one first tissue layer, information on a geometry 21 of the at least one first tissue layer and a 3D map of speed of ultrasounds in the at least one first tissue layer and the volume of at least one second tissue 22 (step 41),

- obtaining the first current position $P_{3D}^{1}$ (step 42),
- carrying out localization computation loop using as starting point the first current position (step 43),
- obtaining the final estimation of the position 31 of the medical device M (step 44).

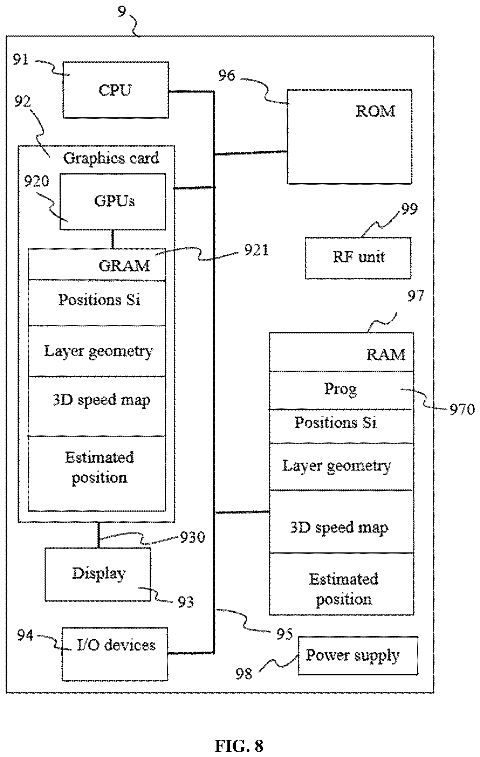

A particular apparatus 9, visible on Figure 8, is embodying the tracking system 1 described above. It corresponds for example to a workstation, a laptop, a tablet, a smartphone, or a head-mounted display (HMD).

That apparatus 9 is suited for estimating a 3D position of a medical device inside the body of a patient.

It comprises the following elements, connected to each other by a bus 95 of addresses and data that also transports a clock signal:

- a microprocessor 91 (or CPU);
- a graphics card 92 comprising several Graphical Processing Units (or GPUs) 920 and a Graphical Random Access Memory (GRAM) 921; the GPUs are quite suited to image processing, due to their highly parallel structure;
- a non-volatile memory of ROM type 96;
- a RAM 97;
- one or several I/O (Input/Output) devices 94 such as for example a keyboard, a mouse, a trackball, a webcam; other modes for introduction of commands such as for example vocal recognition are also possible;
- a power source 98; and
- a radiofrequency unit 99.

According to a variant, the power supply 98 is external to the apparatus 9.

The apparatus 9 also comprises a display device 93 of display screen type directly connected to the graphics card 92 to display synthesized images of the trajectory of the medical device calculated and composed in the graphics card. The use of a dedicated bus to connect the display device 93 to the graphics card 92 offers the advantage of having much greater data transmission bitrates and thus reducing the latency time for the displaying of images composed by the graphics card. According to a variant, a display device is external to apparatus 9 and is connected thereto by a cable or wirelessly for transmitting the display signals. The apparatus 9, for example through the graphics card 92, comprises an interface for transmission or connection adapted to transmit a display signal to an external display means such as for example an LCD or plasma screen or a videoprojector. In this respect, the RF unit 99 can be used for wireless transmissions.

It is noted that the word "register" used hereinafter in the description of memories 97 and 921 can designate in each of the memories mentioned, a memory zone of low capacity (some binary data) as well as a memory zone of large capacity (enabling a whole program to be stored or all or part of the data representative of data calculated or to be displayed). Also, the registers represented for the RAM 97 and the GRAM 921 can be arranged and constituted in any manner, and each of them does not necessarily correspond to adjacent memory locations and can be distributed otherwise (which covers notably the situation in which one register includes several smaller registers).

When switched-on, the microprocessor 91 loads and executes the instructions of the program contained in the RAM 97.

As will be understood by a skilled person, the presence of the graphics card 92 is not mandatory, and can be replaced with entire CPU processing and/or simpler visualization implementations.

**Claims**

1. A tracking system (1) for estimating a 3D position of a medical device (M), equipped of at least one ultrasound sensor, inside an anatomical region of a subject comprising at least one layer (Li) of a first tissue type at least partially surrounding a volume (V) comprising at least one second tissue type, wherein the properties of ultrasounds propagation in the first and second tissue type are different, said system (1) comprising:

   - at least one input configured to receive a position (20) for each of at least three external ultrasound sensors (Si) with respect to the at least one first tissue layer, information on a geometry (21) of the at least one first tissue layer and a 3D map of speed of ultrasounds in the at least one first tissue layer and the volume of at least one second tissue (22);
   - at least one processor configured to:

      o obtain a current position $(P_{3D}^{j})$ of the medical device (M) inside the volume of at least one second tissue;
      o perform location computation comprising:

         ▪ using the obtained current position $(P_{3D}^{j})$ to calculate a current elevation angle $(\theta_i^j)$ and a current lateral angle $(\varphi_i^j)$ of the medical device (M) with respect to each external ultrasound sensor (Si), and using said current elevation angle $(\theta_i^j)$ and current lateral angle $(\varphi_i^j)$ to estimate a current path $(l_i^j)$ traveled by the ultrasounds through the at least one first tissue layer between each external ultrasound sensor (Si) and the medical device (M)
         ▪ for each external ultrasound sensor (Si), obtaining an associated current local speed in the first tissue $(c_i^j)$ using the estimated current path $(l_i^j)$ and

said 3D map of speed in the first tissue;

- calculating a current distance $(d_i^j)$ between each external ultrasound sensor (Si) and the medical device (M) by considering the current local speed in the at least one first tissue layer $(c_i^j)$ associated to the corresponding external ultrasound sensor (Si) and the estimated current path $(l_i^j)$ associated to the corresponding external ultrasound sensor (Si), a speed of ultrasounds in the at least one second tissue and a measured time of propagation of the ultrasounds between each external ultrasound sensor (Si) and the medical device (M);

- estimating a new position of the medical device (M) inside the volume of at least one second tissue using the current distance $(d_i^j)$ between each external ultrasound sensor (Si) and the medical device (M);

o repeat performing location computation using the new position as current position $(P_{3D}^{j+1})$, until an exit criterion is satisfied;

o at least one output configured to provide the estimated position of the medical device (M) satisfying the exit criterion (31).

2. The system according to claim **1**, wherein calculating the current distance $(d_i^j)$ comprises using numerical simulations of the propagation of the ultrasounds in the at least one first tissue layer and the volume of at least one second tissue of the subject, said numerical simulations being based on medical imaging data of at least one portion of the anatomical region of the subject.

3. The system according to either claim **1** or **2**, wherein obtaining a current position $(P_{3D}^j)$ of the medical device (M) inside the volume of second tissue for the first iteration comprises:

- obtaining a thickness $(t_i^1)$ of the at least one first tissue layer at the position of each external ultrasound sensor (Si) using the 3D geometry of the at least one first tissue layer of the subject;

- for each external ultrasound sensors (Si), using the 3D map of speed in the first tissue to obtain a local speed in the first tissue $(c_i^1)$, corresponding to the speed of propagation of ultrasounds through the at least one first tissue layer in correspondence to the external ultrasound sensor position;

- for each external ultrasound sensor (Si), estimating a first distance $(d_i^1)$ between the external ultrasound sensor (Si) and the medical device (M) based on the corresponding obtained local speed in the at least one first tissue layer $(c_i^1)$ propagating through the at least one first tissue layer for a path equal to the corresponding obtained thickness $(t_i^1)$, the speed of ultrasound in the second tissue and a measured time of propagation of the ultrasounds between each external ultrasound sensor (Si) and the medical device (M);

wherein the current position $(P_{3D}^j)$ of the medical device (M) inside the volume of at least one second tissue, for the first iteration, is obtained using the estimated first distances $(d_i^1)$ between the medical device (M) and each external ultrasound sensor (Si).

4. The system according to either one of claims **1** or **2**, wherein obtaining a current position $(P_{3D}^j)$ of the medical device (M) inside the volume of at least one second, for the first iteration, comprises using medical imaging data.

5. The system according to any one of claims **1** to **4**, wherein the measured time of propagation of the ultrasounds between each external ultrasound sensor (Si) and the medical device (M) is based on a direct time of flight measure measured using each external ultrasound sensor (Si) and the at least one ultrasound sensor of the medical device (M).

6. The system according to any one of claims **1** to **5**, wherein the exit criterion is configured to stop the iterations when for a given number of iterations the difference between the current position $(P_{3D}^j)$ and the estimated new position is inferior to a predefined threshold.

**7.** The system according to any one of claims **1** to **6**, wherein the external ultrasound sensors (Si) are ultrasound emitters configured to modify the direction of emission of the ultrasounds and the medical device is equipped with an ultrasound receiver, and wherein the at least one processor is further configured to use the estimated position of the medical device (31) so as to modify the direction of emission of the ultrasounds in order to focus the ultrasounds of the at least three external ultrasound sensors (Si) in the position of the medical device (M).

**8.** The system according to any one of claims **1** to **7**, wherein the at least one layer of a first tissue type is a skull of the subject and the volume of at least on second tissue is a brain of the subject, or the at least one layer of a first tissue type is layer of fat and the at least one volume is a liver.

**9.** A computer-implemented method for estimation of a 3D position of a medical device (M), equipped of at least one ultrasound sensor, inside an anatomical region of a subject comprising at least one layer of a first tissue type at least partially surrounding a volume comprising at least one second tissue type, wherein the properties of ultrasounds propagation in the first and second tissue type are different, said system (1) comprising:

- receiving a position (20) for each of at least three external ultrasound sensors (Si) with respect to the at least one first tissue layer, information on a geometry (21) of the at least one first tissue layer and a 3D map of speed of ultrasounds in the at least one first tissue layer and the volume of at least one second tissue (22);

- obtaining a current position $(P_{3D}^j)$ of the medical device (M) inside the volume of second tissue;

- performing location computation comprising:

▪ using the obtained current position $(P_{3D}^j)$ to calculate a current elevation angle $(\theta_i^j)$ and a current lateral angle $(\varphi_i^j)$ of the medical device (2) with respect to each external ultrasound sensor (Si), and use said current elevation angle $(\theta_i^j)$ and current lateral angle $(\varphi_i^j)$ to

estimate a current path $(l_i^j)$ traveled by the ultrasounds through the at least one first tissue layer between each external ultrasound sensor (Si) and the medical device (2)

▪ for each external ultrasound sensor (Si), estimating an associated current local speed in the first tissue $(c_i^j)$ using the current path $(l_i^j)$ and said 3D map of speed in the first tissue;

▪ calculating a current distance $(d_i^j)$ between each external ultrasound sensor (Si) and the medical device (M) by considering the current local speed in the at least one first tissue layer $(c_i^j)$ associated to the corresponding external ultrasound sensor (Si) and the estimated current path $(l_i^j)$ associated to the corresponding external ultrasound sensor (Si), a speed of ultrasounds in the at least one second tissue and a measured time of propagation of the ultrasounds between each external ultrasound sensor (Si) and the medical device (M);

▪ estimating a new position of the medical device (M) inside the volume of at least one second tissue using the current distance $(d_i^j)$ between each external ultrasound sensor (Si) and the medical device (M);

- repeat performing location computation using the new position as current position $(P_{3D}^{j+1})$, until an exit criterion has been satisfied.

**10.** The method according to claim **9**, wherein calculating the current distance $(d_i^j)$ comprises using numerical simulations of the propagation of the ultrasounds in the first type tissue and second type tissues of the subject, said numerical simulations being based on medical imaging data of at least one portion of the anatomical region of the subject.

**11.** The method according to either one of claims **9** or

10, wherein obtaining a current position $\left(P_{3D}^{j}\right)$ of the medical device (M) inside the volume of second tissue comprises:

- obtaining a thickness $\left(t_{i}^{1}\right)$ of the at least one first tissue layer at the position of each external ultrasound sensor (Si) using the 3D geometry of the at least one first tissue layer of the subject;
- for each external ultrasound sensors (Si), using the 3D map of speed first tissue to obtain a local speed in the first tissue $\left(c_{i}^{1}\right)$, corresponding to the speed of propagation of ultrasounds through the at least one first tissue layer in correspondence to the external ultrasound sensor position;
- for each external ultrasound sensor (Si), estimating a first distance $\left(d_{i}^{1}\right)$ between the external ultrasound sensor (Si) and the medical device (M) based on the corresponding obtained local speed in the first tissue $\left(c_{i}^{1}\right)$ propagating through the at least one first tissue layer for a path equal to the corresponding obtained thickness $\left(t_{i}^{1}\right)$, the speed of ultrasound in the second tissue and a measured time of propagation of the ultrasounds between each external ultrasound sensor (Si) and the medical device (M);

wherein the current position $\left(P_{3D}^{j}\right)$ of the medical device (M) inside the volume of at least one second tissue is obtained using the estimated first distances $\left(d_{i}^{1}\right)$ between the medical device (M) and each external ultrasound sensor (Si).

12. The method according to any one of claims **9** to **11**, wherein the exit criterion is configured to stop the iterations when for a given number of iterations the difference between the current position $\left(P_{3D}^{j}\right)$ and the new position $\left(P_{3D}^{j+1}\right)$ is inferior to a predefined threshold.

13. The method according to any one of claims **9** to **12**, wherein the external ultrasound sensors (Si) are ultrasound emitters configured to modify the direction of emission of the ultrasounds and the medical device is equipped with an ultrasound receiver, and wherein the at least one processor is further configured to use the new position $\left(P_{3D}^{j}\right)$ so as to modify the direction of emission of the ultrasounds in order to focus the ultrasounds of the at least three skull ultrasound sensors (Si) in the position of the medical device (M).

14. A computer program product comprising instructions which, when the program is executed by a computer, cause the computer to carry out the method according to any one of claims **9** to **13**.

15. A computer-readable medium comprising instructions which, when executed by a computer, cause the computer to carry out the method according to any one of claims **9** to **13**.

**FIG. 1**

**FIG. 2**

**FIG. 3**

**FIG. 4**

**FIG. 5**

**FIG. 6**

20    21    22

| Positions Si | Layer geometry | 3D speed map |

Receiving   41

Obtaining first current position   42

Localisation computation loop   43

Obtaining estimated position   44

Estimated position   31

**FIG. 7**

9

91 — CPU

92 — Graphics card

96 — ROM

920 — GPUs

921 — GRAM

99 — RF unit

Positions Si

Layer geometry

3D speed map

Estimated position

97 — RAM

970 — Prog

Positions Si

Layer geometry

3D speed map

Estimated position

930 — Display

93

94 — I/O devices

95

98 — Power supply

**FIG. 8**

20

Europäisches
Patentamt

European
Patent Office

Office européen
des brevets

# EUROPEAN SEARCH REPORT

Application Number

EP 22 30 5850

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2022/000454 A1 (DUPLAT BERTRAND [FR] ET AL) 6 January 2022 (2022-01-06)<br>* abstract *<br>* figures 1-2 *<br>* paragraph [0005] – paragraph [0078] *<br>----- | 1-15 | INV.<br>A61B8/08<br>A61B8/12<br>A61B8/00 |
| A | US 2021/251697 A1 (VAIDYA KUNAL [US] ET AL) 19 August 2021 (2021-08-19)<br>* abstract *<br>* figures 1-8 *<br>* paragraph [0004] – paragraph [0096] *<br>----- | 1-15 | |
| A | US 2018/279996 A1 (COX JEREMY B [US] ET AL) 4 October 2018 (2018-10-04)<br>* abstract *<br>* figures 1-26 *<br>* paragraph [0005] – paragraph [0223] *<br>----- | 1-15 | |

TECHNICAL FIELDS
SEARCHED (IPC)

A61B

The present search report has been drawn up for all claims

| Place of search | Date of completion of the search | Examiner |
|---|---|---|
| Munich | 23 November 2022 | Moehrs, Sascha |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
..........................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 30 5850

This annex lists the patent family members relating to the patent documents cited in the above-mentioned European search report.
The members are as contained in the European Patent Office EDP file on
The European Patent Office is in no way liable for these particulars which are merely given for the purpose of information.

23-11-2022

| Patent document cited in search report | | Publication date | Patent family member(s) | | Publication date |
|---|---|---|---|---|---|
| US 2022000454 | A1 | 06-01-2022 | EP | 3880081 A1 | 22-09-2021 |
| | | | US | 2022000454 A1 | 06-01-2022 |
| | | | WO | 2020135945 A1 | 02-07-2020 |
| US 2021251697 | A1 | 19-08-2021 | CN | 112601496 A | 02-04-2021 |
| | | | EP | 3840661 A1 | 30-06-2021 |
| | | | JP | 2021534857 A | 16-12-2021 |
| | | | US | 2021251697 A1 | 19-08-2021 |
| | | | WO | 2020038758 A1 | 27-02-2020 |
| US 2018279996 | A1 | 04-10-2018 | CN | 107106124 A | 29-08-2017 |
| | | | CN | 112716521 A | 30-04-2021 |
| | | | EP | 3220829 A2 | 27-09-2017 |
| | | | EP | 4011298 A1 | 15-06-2022 |
| | | | US | 2018279996 A1 | 04-10-2018 |
| | | | US | 2020245973 A1 | 06-08-2020 |
| | | | WO | 2016081321 A2 | 26-05-2016 |

EPO FORM P0459

For more details about this annex : see Official Journal of the European Patent Office, No. 12/82